# FASCICULE DE BREVET EUROPEEN

(11) **EP 0 934 043 B1**
(45) Date de publication et mention de la délivrance du brevet: **04.07.2001**
(21) Numéro de dépôt: 97912238.9
(22) Date de dépôt: 22.10.1997
(51) Int. Cl.: A61F 13/08

(54) **BAS DE CONTENTION**
KOMPRESSIONSSTRUMPF
SUPPORT STOCKING

(30) Priorité: 25.10.1996 FR 9613296
(43) Date de publication de la demande: 11.08.1999
(73) Titulaire: BV Sport, 38600 Fontaine (FR)
(72) Inventeur: Couzan, Serge, 42000 Sainte-Etienne (FR); Prufer, Michael, 06240 Beausoleil (FR)
(74) Mandataire: Perrier, Jean-Pierre
(86) Numéro de dépôt international: FR9701892
(87) Numéro de publication internationale: WO9818418

(56) Documents cités:
- EP-A- 0 553 615
- DE-A- 2 413 332
- DE-A- 3 832 798
- DE-C- 602 653
- GB-A- 1 445 233
- GB-A- 2 213 386
- US-A- 3 386 270

## Description

L'invention est relative à un bas de contention adapté aux sports et aux sportifs.

La contention élastique a prouvé son efficacité dans le traitement de l'insuffisance veineuse et particulièrement dans les stades précoces. Elle permet également de ralentir l'évolution vers l'insuffisance veineuse chronique qui se constitue progressivement et le plus souvent sur plusieurs années.

Le sport est théoriquement bénéfique sur le retour veineux mais uniquement dans certaines conditions, qui se retrouvent rarement dans la pratique courante ou de compétition. Ainsi, le type de sport, son intensité, sa durée, le niveau de préparation physique du sportif, le manque d'entraînement et l'insuffisance des temps de récupération peuvent retentir sur l'état veineux et être responsables d'une baisse de la performance ou de la survenue de blessures, telles que claquages, ruptures musculaires, périosites, tendinites chroniques ou rupture du tendon d'Achille.

Les études sur le rôle du sport et de la contention sur le système veineux sont peu nombreuses et les résultats parfois discordants. Pourtant les principes physiques et les effets hémodynamiques de la contention démontrent ses résultats bénéfiques lorsqu'elle est appliquée aux sportifs pendant et après l'activité physique.

Les contentions élastiques actuellement disponibles, avec une pression mesurable, ne sont réservées que pour les patients présentant une insuffisance veineuse fonctionnelle ou organique. Les pressions ont été adaptées en fonction de paramètres relatifs à l'insuffisance veineuse. Or l'ensemble de ces paramètres et les conditions hémodynamiques sont différents pour les sportifs sans insuffisance veineuse.

Il en est ainsi du bas de contention décrit dans le document GB-A-1 445 233 qui, conçu pour des patients présentant une insuffisance veineuse fonctionnelle ou organique, exerce sur la jambe une pression de contention qui est dégressive de bas en haut, en allant de la cheville au mollet, le pied pouvant être sans contention.

Le document US-A- 3 386 270 décrit une chaussette de soutien exerçant sur toutes les parties de la jambe, y compris le pied, un degré de compression sensiblement uniforme.

La présente invention a pour objet d'adapter les bas de contention, obtenus par différents procédés de tricotage, pour obtenir des pressions spécifiques aux sportifs et à la pratique du sport, tout en respectant leur physiologie spécifique et en restant en accord avec les données hémodynamiques.

Les contentions visant à corriger l'insuffisance veineuse sont constituées par des bas, bas-jarrets ou mi-bas, bas-cuisses et collants tricotés, comportant des fils textiles combinés avec des fils élastiques, guipés ou non, d'origine naturelle ou synthétique. La tension communiquée aux fils élastiques lors du tricotage détermine la pression de contention, c'est-à-dire l'effort de serrage radial sur la jambe. Le tricotage peut être circulaire ou rectiligne et l'élasticité dans un ou plusieurs sens. Ces bas de contention peuvent être fabriqués en tailles standard ou sur mesures.

En pratique, la contention procurée par le bas est définie par une pression sanguine mesurée en mm de Mercure, selon les normes françaises (A.F.N.O.R.), et l'on distingue, entre autres, une force I, générant une pression comprise entre 10 et 15 mm de Mercure, une force II, générant une pression comprise entre 15,1 et 20 mm de Mercure, et une force III, générant une pression comprise entre 20,1 et 36 mm de Mercure. Dans les autres pays européens, ces valeurs diffèrent et sont de 13 à 22 mm de mercure pour la classe I, de 23 à 33 mm de Mercure pour la classe II et de 34 à 47 mm de Mercure pour la classe III.

Dans le bas ou mi-bas corrigeant une insuffisance veineuse, la force de contention est maximale sur le pied et la cheville et va en décroissant jusqu'au mollet ou cuisse. Il en est ainsi dans les bas décrits dans US-A-4 745 917. Ce principe est conduit par le fait que, quelle que soit l'étiologie ou les mécanismes responsables de l'insuffisance veineuse (reflux, stase, hyperpression,...), le retentissement veineux est toujours localisé en premier lieu sur la cheville, le pied ou le tiers inférieur de la jambe. FR-A-1 124 593 décrit un bas qui, précisément, procure une contention maximale sur le tiers inférieur de la jambe.

La détermination des pressions thérapeutiques de contention est difficile car de nombreux facteurs interviennent : la position du corps, les conditions anatomiques et biomécaniques des muscles, articulations, tissus, l'état anatomique des structures veineuses et périveineuses, la force de gravité, les pressions tissulaires, l'hémodynamique et les pressions veineuses en changement de position, à l'effort, selon les variations de pressions cardiaques, abdominales, les vitesses des flux et reflux veineux (profonds, superficiels, intermédiaires,...). Seuls quelques uns sont mesurables.

Des travaux antérieurs ont établi un principe de dégressivité des pressions de contention de haut en bas, la pression maximale étant au niveau de la cheville et la pression minimale (= 0) au niveau du coeur. Cette pression maximale de cheville est calculée à partir des données de la physiologie de l'insuffisance veineuse s'accompagnant de reflux et d'hyperdistensibilité veineuse. Ceci ne peut être applicable aux sportifs non insuffisants veineux dont la physiologie est différente et spécifique.

C'est pourquoi les demandeurs ont cherché à définir de nouvelles pressions sanguines et de nouveaux gradients à partir de l'étude de la physiologie du retour veineux et de la contention chez les sportifs non insuffisants veineux, pendant et après l'effort.

Des rappels de physiopathologie paraissent indispensables pour expliquer les éléments déterminants de la présente invention.

Les principaux paramètres physiologiques intervenant chez les sportifs clans la pratique du sport sont représentés par l'hyperdistensibilité veineuse avec augmentation du volume résiduel pauvre en oxygène (jusqu'à 50 % du volume de base) et augmentation du calibre des veines (30 à 40 % pour des efforts longs ou intenses) et par l'augmentation de la pression veineuse transmurale. Les résultats de la littérature sur les mesures des pressions et des variations des pressions veineuses au cours ou après l'effort sont discordants selon la méthodologie, car, le plus souvent, les mesures sont réalisées au niveau de la cheville. Ceci ne peut correspondre à la pression maximale car les sportifs, non insuffisants veineux, ne présentent pas de reflux valvulaire, ont un bon jeu articulaire et une pompe veineuse plantaire et musculaire efficace. Le remplissage et la dilatation veineuse vont d'abord intéresser les muscles du mollet et du réseau veineux superficiel puis les perforantes et le réseau profond. L'hyperpression veineuse va donc être maximale au niveau du mollet.

Il est également intéressant d'examiner la physiologie des veines plantaires, appliquée aux sportifs.

Les axes veineux plantaires sont directement branchés sur les veines tibiales postérieures et elles communiquent également avec les veines superficielles du clos du pied par des perforantes dont certaines de gros calibre. Le réservoir veineux plantaire éjecté à chaque appui peut être estimé entre 30 et 40 ml. Lors de la diastole, trois mécanismes anti-reflux sont mis en jeu. Les valvules, qui sont nombreuses sur les confluents et les collecteurs, un dispositif plexique des confluents veineux qui réalise un véritable barrage et un tunnel fibreux qui entoure les veines les plus exposées au reflux.

Ainsi, ce dispositif anti-reflux est efficace chez les sportifs ne présentant pas d'insuffisance veineuse par incontinence valvulaire. Il n'y a donc pas nécessité de trop comprimer le pied et la région malléolaire chez les sportifs. Ceci trouve un intérêt sur le plan physique (Loi de LAPLACE) mais également sur la tolérance de la contention, la compression du pied et des malléoles étant la plus mal supportée. A cela, il faut ajouter que, avec l'amélioration des caractéristiques podologiques de la plupart des chaussures de sport, les conditions d'éjection du réservoir veineux plantaire sont optimales.

Le bas de contention pour la pratique des sports, selon l'invention, est composé d'une enveloppe qui, s'étendant du pied à la jambe et élastiquement déformable au moins dans un sens, est conformée, lors de la réalisation du bas, par exemple par couture de ses bords ou tricotage circulaire, de manière à exercer sur le corps, c'est-à-dire au moins sur le pied et la jambe, des pressions de contention qui sont différentes entre le pied et la jambe.

Selon l'invention, la partie du bas, s'étendant de l'extrémité du pied jusqu'au dessus des malléoles et enveloppant le pied et la cheville, est structurée pour exercer une pression de contention P1 de valeur inférieure à celle de la pression P2, exercée par la partie du bas enveloppant le mollet, cette pression étant dégressive du haut vers le bas, c'est-à-dire en allant du mollet vers le pied, entre les valeurs P2 et P1.

Avec ce bas, la contention exercée sur un sportif est donc modérée au niveau du pied jusqu'aux malléoles et plus importante sur le mollet, et va donc à contre courant de la contention utilisée pour le traitement des insuffisances veineuses qui, elle, va en décroissant en allant du pied vers le haut, en serrant fortement ce pied et la cheville, où elle est maximale.

Cette contention appliquée aux sportifs procure les effets hémodynamiques suivants :
a) amélioration de la fonction de la pompe veineuse du mollet par augmentation du volume de chasse veineuse de 20 à 50 %, diminution du volume résiduel, augmentation du temps de remplissage veineux après exercice et amélioration du remplissage cardiaque droit,
b) diminution de la pression veineuse et augmentation de la vitesse du flux veineux entraînant un effet de décongestion des muscles et des tissus,
c) diminution du calibre des veines superficielles et profondes favorisant le rapprochement des valvules écartées lors de la distension de la paroi survenant à l'effort. Ceci donne la possibilité de restauration de la fonction valvulaire incompétente. Les conséquences sont la diminution de l'accumulation du sang veineux pauvre en oxygène dans le mollet réduisant la stase et l'hypoxie et favorisant l'élimination des déchets. Il se produit un effet métabolique avec une meilleure oxygénation musculaire, tendineuse et tissulaire diminuant ainsi le risque d'inflammation et toutes ses conséquences pathogènes.
d) les effets sur la microcirculation sont représentés par une augmentation du débit cutané et une amélioration du réflexe veino-artériolaire. Il a également été constaté la possibilité de régression des altérations pariétales et de réversibilité de la médiadysplasie lors de la mise en place précoce de la contention.
e) Le port régulier d'une contention fait bénéficier le sportif d'un effet de rémanence procurant une persistance des effets hémodynamiques, après abandon de cette contention, comme cela a été constaté après le port continu d'une contention pour insuffisance veineuse.

Dans une forme d'exécution de l'invention, et lorsque l'enveloppe contient des fils élastiques dont la tension au tricotage détermine la pression de contention, chaque bas est divisé, par une ligne fictive, allant du tendon d'Achille au coup de pied et passant au-dessus des malléoles, en une partie inférieure, enveloppant le pied et la cheville et dans laquelle les fils élastiques exercent une tension procurant une pression P1, et une partie supérieure, s'étendant du dessus des malléoles jusqu'au dessous du genou et enveloppant le mollet et la jambe dans laquelle les fils élastiques exercent une tension procurant, au moins sur le mollet, une pression P2 supérieure à la pression P1.

Dans le bas selon l'invention, la force de pression maximale est localisée au niveau du mollet. En effet, et contrairement aux bas de contention pour les insuffisants veineux, la pression à la partie supérieure du mollet est suffisamment forte pour agir sur le réseau veineux superficiel, musculaire et profond, sans exercer d'effet garrot ou occlusif. Ceci est particulièrement important pour la contention spécifique « sur mesures » appliquée aux sportifs de haut niveau, car il faut obtenir, au maximum par « l'effet aponévrose », le rôle de muscle supplémentaire améliorant la performance, et par "effet turbo", la meilleure oxygénation de la fibre musculaire, pendant et après l'effort, permettant de faciliter la récupération et de diminuer le risque d'inflammation.

Cependant, pour les sportifs de base et pour faciliter la fabrication sur les machines de tricotage, il est possible de maintenir une dégressivité du haut vers le bas, mais en conservant toujours, au niveau du mollet, une pression supérieure à la pression exercée sur la cheville et le pied, ce qui différencie la contention du sportif de la contention de l'insuffisant veineux.

La caractéristique principale de l'invention est d'appliquer aux bas de contention des sportifs des pressions et gradients de pressions différents et opposés aux bas des insuffisants veineux, sans produire d'effet garrot.

Lorsqu'un tel bas est porté, la contention élastique incluse dans le bas agit en compression et favorise le retour veineux et les mouvements liquidiens vers le coeur gauche, diminue la stase veineuse, facilite l'élimination des métabolites acides et permet ainsi une meilleure oxygénation de la fibre musculaire (effet turbo). L'association de la contention et de la compression trouve sa pleine efficacité lors de l'action musculaire, car elle renforce les effets de la contraction musculaire, en se comportant comme un muscle supplémentaire (effet aponévrose). D'autres modes particuliers selon l'invention sont indiqués dans les revendications 3, 4.

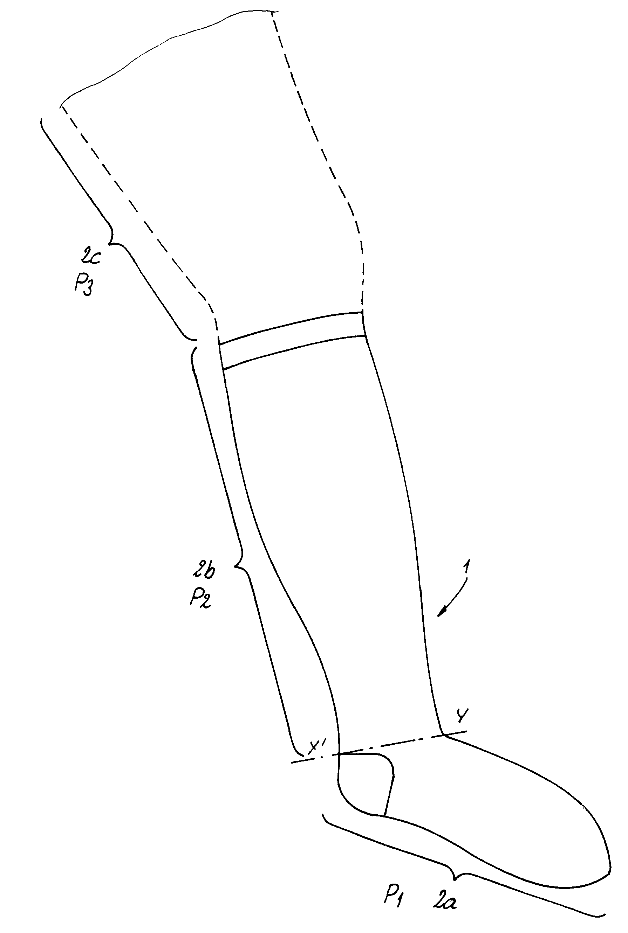

D'autres caractéristiques et avantages ressortiront de la description qui suit en référence au dessin schématique annexé, dont l'unique figure représente, vue de côté, un bas-jarret selon l'invention, dont l'enveloppe est obtenue par tricotage.

Ce bas-jarret 1 est divisé de part et d'autre de la ligne y'-y, représentée en traits mixtes, allant du tendon d'Achille au coup de pied en passant au-dessus des malléoles, d'une part, en une partie inférieure 2a enveloppant le pied et la cheville et, d'autre part, en une partie supérieure 2b s'arrêtant sous le genou et enveloppant la jambe et surtout le mollet. Dans les cas de bas-cuisse ou collant la partie 2b est prolongée par une partie supplémentaire 2c enveloppant la cuisse. Ces parties qui, de façon connue, sont réalisées par tricotage sur un métier maille suivant une armure et des conditions bien connues de l'homme de l'art fabriquant des bas de contention pour le traitement des insuffisances veineuses, se différencient de ces derniers par les valeurs de la tension donnée aux fils élastiques déterminant la contention. Tout procédé de tricotage ou tissage et tous matériaux pouvant transmettre ces valeurs de tension peuvent être utilisés.

Dans une forme d'exécution de l'invention, visant plus spécialement les sports à faibles risques veineux et les sportifs de niveaux bas et moyen, les valeurs des pressions de contention, respectivement P1 dans la partie inférieure 2a, P2 dans la partie supérieure 2b, et éventuellement P3 dans la partie supplémentaire 2c sont indiquées dans le tableau ci-dessous.

| | Partie inférieure 2a P1 | Partie supérieure 2b P2 | Partie supplémentaire 2c P3 |
|---|---|---|---|
| Valeur de la pression en mm de Hg | 7 à 13 | 15 à 25 | 7 à 13 |
| en Pascal | 920 à 1710 | 1973 à 3289 | 920 à 1710 |
| Valeur de référence en mm de Hg | 10 | 20 | 10 |
| en Pascal | 1315 | 2631 | 1315 |

On notera que, dans le bas-cuisse ou le collant, la valeur P3 est du même ordre que la valeur de P1.

Pour les sportifs de haut niveau ou les sports à risque veineux exerçant des contraintes importantes sur le retour veineux, de type accélérations ou décélérations brutales, (automobile, formule 1, voltige aérienne,...) ou valsalva prolongé (haltérophilie, musculation,...), les pressions de contention sont déterminées cas par cas. Dans cette forme d'exécution de l'invention, la tension communiquée aux fils élastiques conserve les gradients de valeurs selon l'invention avec des pressions P1 au niveau du pied et de la cheville dans la partie 2a, toujours inférieures à celles P2 appliquées au niveau du mollet. Dans le cas de bas-cuisses ou collants, la tension communiquée aux fils élastiques dans la partie supplémentaire 2c fournit une pression de contention P3 identique ou similaire à celle P1 sur le pied et la cheville, et en tous cas inférieure à P2. Pour ne pas exercer d'effet garrot, dans cette application spécifique aux sportifs de haut niveau, la pression P2 exercée au niveau du mollet ne doit pas être supérieure au triple de la valeur de la pression P1 exercée au niveau du pied et de la cheville.

Dans une variante, non représentée, chaque bas est réalisé à partir de nappes extensibles simples ou complexes qui sont structurées, c'est-à-dire découpées et cousues ou collées les unes aux autres pour former une enveloppe comportant les parties 2a, 2b et éventuellement 2c procurant, lorsqu'elles sont sur le corps, les pressions de contention définies ci-dessus.

Le bas selon l'invention doit, bien entendu, être porté pendant l'effort et pris immédiatement après l'effort pour améliorer la récupération, et cela surtout en cas de transport prolongé ou piétinement après l'effort.

Dans certains cas standard, en fonction de la tolérance, la contention différentielle procurée par chaque bas est doublée, après l'effort, par mise en place sur le premier bas d'un second bas identique au premier, ce qui accélère la récupération.

Une étude clinique réalisée sur des sportifs ayant utilisé de manière confidentielle des bas-jarrets selon l'invention a montré que les résultats procurés par ce bas-jarret étaient bénéfiques après l'effort, pendant la période de récupération, mais aussi au cours de l'effort.

Pendant l'effort, tous les signes cliniques ont régressé et plus particulièrement l'oedème. Pour les autres signes, les améliorations sont les suivantes :
- lourdeur ou fatigabilité 86 %,
- crampes 89 %,
- dysesthésies 71 %.

La contention a également réduit de manière significative la durée des signes cliniques et a facilité la récupération. Chez les sportifs pratiquant des épreuves chronométrées ou chiffrées, l'amélioration de la performance physique a été constatée clans 88 % des cas. La contention a été jugée bénéfique sur des douleurs séquellaires d'inflammation ou d'accidents musculaires ou tendineux dans 83 % des cas.

Après l'effort, l'efficacité de la contention a été maximale. Cette période qui, en théorie, correspond à une phase de récupération est négligée par la plupart des sportifs. Très souvent, les étirements ou les massages ne sont ou ne peuvent être réalisés et la période qui suit l'effort aggrave la stase veineuse (piétinement, station assise prolongée, ...). La contention, bien qu'elle ne remplace pas ces techniques de récupération, en est un excellent complément et est équivalente à une séance de pressothérapie ou à une nuit de repos, lorsqu'elle est portée au moins deux heures après l'effort. Dans le cadre de cette étude, il s'est révélé que tous les signes cliniques ont été améliorés par le port de la contention pendant cette phase de récupération. Les plus fréquents étaient la sensation de lourdeur ou de fatigabilité qui a disparu dans 97 % des cas, les impatiences dans 93 % des cas, les crampes dans 98 % des cas, les dysesthésies dans 100 % des cas. De manière moins significative, car peu gênantes et trop rares sur l'échantillon examiné, des améliorations ont été constatées sur l'oedème et les phlébalgies.

Dans la plupart des cas standard, une meilleure récupération a été apportée lorsque, après l'effort, le sportif passait, sur chaque jambe, un second bas-jarret identique au premier, ce qui avait pour effet de réduire le volume de stase, de réduire le calibre de toutes les veines en accélérant de façon significative le débit de retour, et en conséquence, l'évacuation du sang pauvre en oxygène.

Bien que très intéressant pour les sportifs de haut niveau, le bas selon l'invention vise aussi le sportif courant et voire même occasionnel car, tout en améliorant les performances et la récupération musculaire (avec réduction des périodes de fatigue), il repousse dans le temps les risques veineux. Dans les sports définis comme entraînant ce risque, il compense les insuffisances veineuses, connues ou encore ignorées, et retarde la maladie veineuse.

## Revendications

1. Bas de contention adaptés aux sports et aux sportifs, composé d'une enveloppe s'étendant du pied à la jambe, étant élastiquement déformable, au moins dans un sens, et étant, lors de la confection du bas, conformée pour exercer sur le corps une pression de contention ayant des valeurs différentes sur le pied et sur la jambe, **caractérisé en ce que** la partie (2a) du bas, s'étendant de l'extrémité du pied jusqu'au dessus des malléoles et enveloppant le pied et la cheville, est structurée pour exercer une pression de contention (P1) de valeur inférieure à celle de la pression de contention (P2) exercée par la partie (2b) du bas enveloppant le mollet, cette pression de contention étant dégressive du mollet vers le bas entre les valeurs (P2) et (P1).

2. Bas selon la revendication 1, **caractérisé en ce que,** lorsque l'enveloppe contient des fils élastiques dont la tension au tricotage détermine la pression de contention, chaque bas est divisé, par une ligne fictive (y'-y), allant du tendon d'Achille au coup de pied et passant au-dessus des malléoles, en une partie inférieure (2a), enveloppant le pied et la cheville et dans laquelle les fils élastiques exercent une tension procurant une pression (P1), et une partie supérieure (2b), s'étendant du dessus des malléoles jusqu'au dessous du genou et enveloppant le mollet et la jambe dans laquelle les fils élastiques exercent une tension procurant, au moins sur le mollet, une pression (P2) supérieure à la pression (P1).

3. Bas selon la revendication 1, **caractérisé en ce que,** lorsque le bas comporte une partie supplémentaire (2c) prolongeant la partie (2b) et s'étendant sur la cuisse, la tension communiquée aux fils élastiques de cette partie (2c) procure une pression de contention (P3) ayant une valeur de même ordre que celle de la pression (P1) exercée sur la partie inférieure (2a).

4. Bas selon la revendication 1, **caractérisé en ce que** la pression de contention (P1) a une valeur comprise entre 920 et 1710 Pascal, tandis que la pression de contention (P2) exercée sur le mollet et sur la partie supérieure (2b) a une valeur comprise entre 1973 et 3289 Pascal.

## Patentansprüche

1. Stützstrumpf für Sport und Sportler, bestehend aus einer Umhüllung, die sich vom Fuß zum Bein erstreckt und zumindest in einer Richtung elastisch verformbar ist und bei der Anfertigung des Strumpfs geformt wird, um auf den Körper einen Stützdruck mit unterschiedlichen Werten auf Fuß und Bein auszuüben, **dadurch gekennzeichnet, dass** der sich von der Fußspitze bis über die Malleoli erstreckende und den Fuß und den Knöchel umhüllende Teil (2a) des Strumpfs strukturiert ist, um einen Stützdruck (P1) mit einem niedrigeren Wert als der des Stützdrucks (P2), der durch den die Wade umhüllenden Teil (2b) des Strumpfs ausgeübt wird, auszuüben, wobei der Stützdruck von der Wade nach unten zwischen den Werten (P2) und (P1) abfällt.

2. Strumpf nach Anspruch 1, **dadurch gekennzeichnet, dass,** wenn die Umhüllung elastische Fäden enthält, deren Spannung beim Stricken bzw. Wirken den Stützdruck bestimmt, jeder Strumpf durch eine sich von der Achillessehne zum Fußblatt erstreckende und oberhalb der Malleoli verlaufende fiktive Linie (y'-y) in einen den Fuß und den Knöchel umhüllenden unteren Teil (2a), in welchem die elastischen Fäden eine einen Druck (P1) bewirkende Spannung ausüben, und einen sich von oberhalb der Malleoli bis unterhalb des Knies erstreckenden und die Wade und das Bein umhüllenden oberen Teil (2b), in welchem die elastischen Fäden eine Spannung ausüben, die, zumindest auf die Wade, einen Druck (P2) bewirkt, der größer ist als der Druck (P1), geteilt ist.

3. Strumpf nach Anspruch 1, **dadurch gekennzeichnet, dass,** wenn der Strumpf einen zusätzlichen den Teil (2b) verlängernden und sich auf dem Oberschenkel erstreckenden Teil (2c) aufweist, die den elastischen Fäden des Teils (2c) verliehene Spannung einen Stützdruck (P3) bewirkt mit einem Wert derselben Größenordnung wie der des auf den unteren Teil (2a) ausgeübten Drucks (P1).

4. Strumpf nach Anspruch 1, **dadurch gekennzeichnet, dass** der Stützdruck (P1) einen zwischen 920 und 1710 Pascal enthaltenen Wert aufweist, während der auf die Wade und den oberen Teil (2b) ausgeübte Stützdruck (P2) einen zwischen 1973 und 3289 Pascal enthaltenen Wert aufweist.

## Claims

1. A support stocking adapted to sports and sportsmen, comprising a covering extending from the foot to the leg, being elastically deformable in at least one direction and, during manufacture of the stocking, being shaped so as to exert on the body a support pressure having different values over the foot and the leg, characterised in that the part (2a) of the stocking extending from the extremity of the foot to above the malleoli and covering the foot and the ankle is structured so as to exert a support pressure (P1) having a lower value than that of the support pressure (P2) exerted by the part (2b) of the stocking covering the calf, this support pressure decreasing between the values (P2) and (P1) from the calf towards the foot.

2. A stocking according to claim 1, characterised in that, when the covering comprises elastic threads, the knitting tension of which determines the support pressure, each stocking is divided by an imaginary line (y'-y), running from the Achilles tendon to the front of the foot and extending above the malleoli, into a lower part (2a) which covers the foot and the ankle and in which the elastic threads exert a tension producing a pressure (P1), and an upper part (2b) which extends from above the malleoli until below the knee and covers the calf and the shin and in which the elastic threads exert a tension producing, at least on the calf, a pressure (P2) greater than the pressure (P1).

3. A stocking according to claim 1, characterised in that, when the stocking comprises an additional part (2c) extending the part (2b) and covering the thigh, the tension imparted to the elastic threads of this part (2c) produces a support pressure (P3) having a value of the same order as that of the pressure (P1) exerted on the lower part (2a).

4. A stocking according to claim 1, characterised in that the support pressure (P1) has a value between 920 Pa and 1710 Pa, whereas the support pressure (P2) exerted on the calf and the upper part (2b) has a value between 1973 Pa and 3289 Pa.
